# EUROPEAN PATENT SPECIFICATION

(11) **EP 3 303 276 B1**
(45) Date of publication and mention of the grant of the patent: **12.04.2023**
(21) Application number: 16739394.1
(22) Date of filing: 27.05.2016
(51) Int. Cl.: C07C 37/00, C07C 37/055, C07C 39/06

(54) **PRODUCTION OF 3-ALKYLPHENOLS**
HERSTELLUNG VON 3-ALKYLPHENOLEN
PRODUCTION DE 3-ALKYLPHÉNOLS

(30) Priority: 28.05.2015 US 201562167807 P
(43) Date of publication of application: 11.04.2018
(73) Proprietor: Katholieke Universiteit Leuven, 3000 Leuven (BE)
(72) Inventor: SCHUTYSER, Wouter, 3940 Hechtel-Eksel (BE); SELS, Bert, 2260 Westerlo (BE); VAN DEN BOSCH, Sander, 3190 Bortmeerbeek (BE)
(74) Representative: IPLodge bv
(86) International application number: PCT/BE2016/000022
(87) International publication number: WO 2016/187678

(56) References cited:
- US-A- 1 300 877
- John H P Tyman ET AL: "The synthesis of phenolic propane-1, 2-and 1, 3-diols as intermediates in immobilised chelatants for the borate anion 1", , 1 January 2006 (2006-01-01), XP55300160, Retrieved from the Internet: URL:http://docserver.ingentaconnect.com/de liver/connect/stl/03082342/v2006n11/s3.pdf ?expires=1473158330&id=88547208&titleid=89 3&accname=European+Patent+Office&checksum= 0A83A9394D9424A288138FDD4CCBD527 [retrieved on 2016-09-06]

## Description

### FIELD OF INVENTION

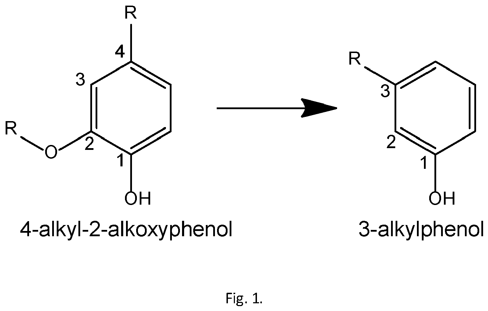

The invention is as defined in the claims; in general it concerns a method for conversion of particular 4-alkyl-2-hydroxyphenols (e.g. 4-*n*-propyl-2-hydroxyphenol, 4-ethyl-2-hydroxyphenol) and 4-alkyl-2-alkoxyphenols (e.g. 4-*n*-propyl-2-methoxyphenol, 4-ethyl-2-methoxyphenol or 4-*n*-propyl-2-ethoxyphenol) into 3-alkylphenols. More specifically, this invention relates to a novel process of selectively forming meta-alkyl phenols of various alkylphenols, such as for instance converting the fraction of 4-alkyl-2-hydroxyphenols and 4-alkyl-2-alkoxyphenols into high yields of 3-alkylphenols (Fig.1).

### BACKGROUND

Lignocellulose biomass is a highly abundant and potentially low cost renewable feedstock to produce heat, power, fuels, chemicals and materials as analternative for fossil resources (M. Dusselier, M. Mascal and B. Sels, in Selective Catalysis for Renewable Feedstocks and Chemicals, ed. K. M. Nicholas, Springer International Publishing, 2014, vol. 353, ch. 544, pp. 1-40; J. S. Luterbacher, D. Martin Alonso and J. A. Dumesic, Green Chem., 2014, 16, 4816-4838). Lignocellulose is composed of three main constituents, cellulose, hemicellulose and lignin. Until recently, lignocellulose biorefineries have mainly focused on the valorization of (hemi)cellulose, while lignin was regarded as a side product and mostly used as energy source (by burning) (J. Zakzeski, et al., Chem Rev., 2010, 110, 3552-3599). Only a minor amount of lignin is used for material applications (dispersants, emulsifiers,...) or in the production of chemicals like vanillin (J. Lora, in Monomers, Polymers and Composites from Renewable Resources, ed. M. N. B. Gandini, Elsevier, Amsterdam, 2008, pp. 225-241; J. Bozell, in Selective Catalysis for Renewable Feedstocks and Chemicals, ed. K. M. Nicholas, Springer International Publishing, 2014, vol. 353, ch. 535, pp. 229-255).

There is however a growing consensus that lignin valorization isessential to the environmental sustainability and economics of a lignocellulosic biorefinery (A. J. Ragauskas, et al., Science, 2014, 344, 709). Lignin constitutes the largest direct source of renewable aromatic/phenolic compounds on Earth and is regarded as a promising feedstock for a wide variety of bulk and fine chemicals, as well as fuels.

The most important lignin depolymerization methods are pyrolysis, base-catalyzed depolymerization, hydrogenolysis, liquid-phase reforming, chemical oxidation and gasification, usually yielding moderate amounts (< 20 wt%) of numerous monomeric compounds (C. Xu, R.A.D. Arancon, J. Labidid and R. Luque, Chem. Soc. Rev. 2014, 43, 7485-7500). Very promising routes for lignin depolymerization are reductive methods like hydrogenolysis and liquid-phase reforming, resulting in phenolic monomer-yields up to 55 wt% (J. M. Pepper and W. Steck, Can. J. Chem., 1963, 41, 2867-2875; J.M. Pepper, P. Supathana, Can. J. Chem., 1978, 56, 899-902; N. Yan, C. Zhao, P. J. Dyson, C. Wang, L. T. Liu and Y. Kou, Chemsuschem, 2008, 1, 626-629; C. Li, M. Zheng, A. Wang and T. Zhang, Energy Environ. Sci., 2012, 5, 6383-6390; Q. Song, F. Wang, J. Y. Cai, Y. H. Wang, J. J. Zhang, W. Q. Yu and J. Xu, Energy Environ. Sci., 2013, 6, 994-1007). The phenolic compounds obtained from these processes possess unique chemical structure features. The phenolic entity bears one or two methoxy groups, next to a 4-alkyl chain. Use of such 4-alkylguaiacols (4-alkyl-2-methoxyphenols) and 4-alkylsyringols (4-alkyl-2,6-dimethoxyphenols) as intermediates for high-value target chemicals and polymer building blocks has thus far received only little attention. Until now, main research goals have focused on upgrading the lignin-derived compounds to hydrocarbon fuels or aromatics by complete removal of oxygen through hydrodeoxygenation (HDO).

A possible processing of the phenolic compounds into useful products is its conversion into alkylphenols. Therefore, the methoxy group(s) should be removed (demethoxylation). Direct demethoxylation yields4-alkylphenols (N. Joshi, A. Lawal, Ind. Eng. Chem. Res. 2013, 52, 4049-4058). However, a challenge is the production of 3-alkylphenols or m-alkylphenols since these are very interesting chemicals used in various applications.

3-ethylphenol is an intermediate for the production of photochemicals and a possible intermediate for the production of pharmaceuticals and agricultural chemicals (Fiege, H., Voges, H.-W., Hamamoto, T., Umemura, S., Iwata, T., Miki, H., Fujita, Y., Buysch, H.-J., Garbe, D. and Paulus, W. 2000. Phenol Derivatives. Ullmann's Encyclopedia of Industrial Chemistry). 3-ethylphenol and 3-n-propylphenol are potent tsetse fly attractants (C. H. Green, in Advances in Parasitology, eds. R. M. J.R. Baker and D. Rollinson, Academic Press, 1994, vol. Volume 34, pp. 229-291; E. Bursell, A.J.E. Gough, P.S. Beevor, A. Cork, D.R. Hall, G.A. Vale, Bull. ent. Res. 1988, 78, 281-291; M.L.A. Owaga, A. Hassanali, P.G. McDowell, Insect Sci. Applic. 1988, 9, 95-100; G.A. Vale, D.R. Hall, A.J.E. Gough, Bull. ent. Res. 1988, 78, 293-300). Tsetse flies are the main vector of African sleeping sickness *(trypanosomiasis),* and therefore several tsetse control methods are developed to monitor, reduce or even eradicate local populations of targeted tsetse species (R. Brun, J. Blum, F. Chappuis, C. Burri, Lancet, 2010, 375, 148-159.; I. Ujváry, G. Mikite, Org. Process Res. Dev., 2003, 7, 585). The use of traps baited with natural or artificial host odours is an environmentally benign tsetse control method. 3-*n*-propylphenol is used extensively in artificial odour baits, mostly together with acetone, *p*-cresol and 1-octen-3-ol. 3-*n*-propylphenol can also be used in antimicrobial compositions for treatment and prevention of disease in mammals (J.G. Holwerda, B.D. Levine, US 20050165104 A1). 3-alkylphenols like 3-methylphenol, 3-ethylphenol and 3-*n*-propylphenol represent leather- and ink-like odor notes, as well as having a medicinal smell (M. Czerny, et al., Chem. Senses, 2011, 539-553). 3-alkylphenols like 3-ethylphenol, 3-*n*-propylphenol and 3-*n*-butylphenol can also be used as perfuming ingredients to improve the odour of perfuming compositions having an oud character (E. Delort, J. Limacher, Oud Odorants, US 20140371121 A1).

3-ethylphenol or *m*-ethylphenol is conventionally produced by sulfonation of ethylbenzene at high temperature (150-210 °C) to form an isomeric mixture of ethylbenzenesulfonic acids rich in the m-isomer (o/m/p ratio: 1.5/58.5/39.2), followed by selective hydrolysis of the o- and p-isomers back to ethylbezene and sulfonic acid by blowing steam through the mixture (o/m/p ratio: 0.8/95.7/2.3), and alkali fusion of the remaining *m*-ethylbenzenesulfonic acid (o/m/p ratio: 0.9/95.7/2.6) (Taoka Chemical Co., EP0080880 / JP 59 108 730 / JP 59 076 033). This process results in a 72% ethylphenol yield on ethylbenzene basis. This process has many problems such as tedious multi-step operation, inferior operational surroundings associated with handling of dangerous high-temperature sulfuric acid and sodium hydroxide, corrosion of equipment caused by the use of sulfuric acid, and disposal of waste water containing sulfuric acid and alkali (Maruzen Petrochemical Co., EP0296582). A variation of the desulfonation step, involving the addition of steam to the mixture of sulfonic acid in an excess in relation to the amount of evaporable water, in which the formed ethylbenzene is removed from the reaction mixture, makes it possible to obtain a higher *m*/*p*-ratio of the ethylphenols (Kemira AgroOy, EP0694516).

3-ethylphenol can also be obtained through alkylation of phenol with ethylene or ethanol over a crystalline aluminosilicate (H-ZSM-5/Al₂O₃) at high temperature (400 °C), yielding an isomeric mixture of ethylphenols rich in the *m*-isomer (S_{o/m/p}: 23.3/39.8/13.1% for ethylene alkylation at 50.2% phenol conversion and 23.8/39.2/13.5% for ethanol alkylation at 48% phenol converson), followed by selective dealkylation of the *o*- and *p*-isomers over a specific crystalline aluminosilicate catalyst (Mg-P-ZSM-5/Al₂O₃ or Si-P-ZSM-5/Al₂O₃) at high temperature (450 °C) (Maruzen Petrochemical Co., EP0296582). Dealkylation with Mg-P-ZSM-5/Al₂O₃ of the *m*/*p*-ethylphenol mixture after ethylene alkylation yields a S_{o/m/p} of 0.1/67.7/0.9% and a *m*-ethylphenol recovery of 90.4%. With Si-P-ZSM-5/Al₂O₃, a S_{o/m/p} of 0.1/68.2/1.0% and a *m*-ethylphenol recovery of 91.1% is obtained.

Mixtures of *m*- and *p*-ethylphenol can be obtained from the alkylation of phenol or from distillation of high-temperature bituminous coal tar. However, since these compounds have very similar boiling points, separation by distillation is not possible (Fiege, H., Voges, H.-W., Hamamoto, T., Umemura, S., Iwata, T., Miki, H., Fujita, Y., Buysch, H.-J., Garbe, D. and Paulus, W. 2000. Phenol Derivatives. Ullmann's Encyclopedia of Industrial Chemistry). Next to the separation method described in EP0296582, other separation methods like those used for the separation of *m*- and *p*-cresol can be applied, e.g. crystallization, adsorption, separation via addition compounds or via ester or salt formation, etc. (Fiege, H. 2000. Cresols and Xylenols. Ullmann's Encyclopedia of Industrial Chemistry).

Several routes are described for the synthesis of 3-*n*-propylphenol: reductive deoxygenation of 3-hydroxypropiophenone (Hartung, W. H.; Crossley, F. S. J. Am. Chem. Soc. 1934, 56, 158.), reductive deoxygenation of safrole or isosafrole (Henrard, J. T. Chem. Zentralbl. 1907(II), 78, 1512.; Cousin, S. G., Lions, F. J. Proc. R. Soc. N. S. W. 1937, 70, 413; Strunz, G. M., Court, A. S. J. Am. Chem. Soc. 1973, 95, 3000.), Wittig reaction of 3-hydroxybenzaldehyde with ethyl(triphenyl)phosphonium bromide (Bursell, E., Gough, A. J. E., Beevor, P. S., Cork, A., Hall, D. R., Vale, G. A. Bull. Entomol. Res. 1988, 78, 281.), C-C coupling of 3-bromoanisole and ethyl halide (Hassanali, A., McDowell, P. G., Owaga, M. L. A., Saini, R. K. Insect Sci. Its Appl. 1986, 7, 5.), cyclocondensation of 3-oxohexanal with 1,3-acetonedicarboxylic acid esters (Prelog, V., Würsch, J., Königsbacher, K. Helv. Chim. Acta 1951, 34, 258.), catalytic carbanionic ethylation of *m*-cresol (Steele, B.R., Villalonga-Barber, C., Micha-Screttas, M., Screttas, C.G., Tetrahedron Lett. 2006, 47, 2093), Grignard reaction of 3-benzyloxybenzaldehyde with ethylmagnesium bromide and subsequent reductive deoxygenation of the secondary alcohol (Carvalho, C. F., Sargent, M. V. J. Chem. Soc., Perkin Trans. 1 1984, 1621.) and Grignard reaction of 3-hydroxybenzaldehyde with ethylmagnesium bromide followed by reductive deoxygenation of the secondary alcohol (I. Ujváry, G. Mikite, Org. Process Res. Dev., 2003, 7, 585). Most of these routes however require expensive or toxic (even forbidden) substrates and include multistep reactions with waste-intensive key steps.

TYMAN, J.H.P. et al, The synthesis of phenolic propane-1, 2- and 1, 3-diols as intermediates in immobilized chelatants for the borate anion, 1 January 2006 (2006-01-01), XP055300160 discloses a 2-step synthesis of an 3-alkylphenol, starting from a 4-alkyl-2alkoxyphenol.

Two recently developed synthesis routes involve anacardicacidderivatives(salicylicacidswith saturated and mono, di an tri-unsaturated C₁₅ side chains in the 6-position) extracted from cashew nut shells as feedstock. In the first synthesis route, the anacardic acid is heated in a graphite bath to produce cardanol (3-(pentadec-8-enyl)phenol), which is subsequently converted via carbon-carbon double bond isomerisation, metathesis with 2-butene and hydrogenation, resulting in a relatively low yield of 3-n-propyphenol of 11% based on cardanol (Mmongoyo, J.A., Mgani, Q.A., Mdachi, S.J.M., Pogorzelec P.J., Cole-Hamilton, D.J., Eur. J. Lipid Sci. Technol., 2012, 114, 1183-1192). In the second synthesis route, anacardic acid is first converted to 3-(non-8-enyl)phenol through ethenolysis and distillation, followed by isomerizing cross-metathesis with short-chain olefins and hydrogenation (S. Baader, P.E. Podsiadly, D.J. Cole-Hamilton, L.J. Goossen, Green Chem. 2014, 16, 4885-4890). In the latter synthesis route, both 3-ethyl- and 3-n-propylphenol can be produced in a combined yield of 85% based on the anacardic acids in a 1:1.3 ratio when ethylene is used during the cross-metathesis. If an extra cross-metathesis step with 2-butene is applied, 3-n-propylphenol can be obtained in a total yield of 69% based on the anacardic acids. An extra cross-metathesis step with ethylene yields 3-ethylphenol in a yield of 84% based on anacardic acids. Although this last synthesis route results in high product yields of the 3-alkylphenols starting from a renewable substrate, the fact that it is a multistep process using expensive Ru- and Pd-based homogeneous catalysts and ethylene, 2-butene and hydrogen as extra reagents hinders its application on a large scale.

A particular embodiment of present invention concerns selective conversion of 4-alkyl-2-alkoxyphenols towards a mixture of 3-alkylphenols like 3-ethylphenol and 3-n-propylphenol.

4-alkyl-2-alkoxyphenol conversion ranges in mole percent are more than 80%, preferably more than 90%. The yield in mole percent for 3-alkylphenols in the range of 50 to 90%, preferably within the range of 60 to 90%.

Next to 3-alkylphenol, also a small amount of 4-alkylphenol can be obtained, but the 3- and 4-alkylphenols can be separated trough the processes described earlier. The selectivity for such 3-alkylphenol in mole percent is typically less than 22%, preferably less than 12%.

### SUMMARY OF THE INVENTION

Certain 4-alkyl-2-hydroxyphenols and 4-alkyl-2-alkoxyphenols like 4-ethyl-2-hydroxyphenol, 4-*n*-propyl-2-hydroxyphenol, 4-ethyl-2-methoxyphenol and 4-*n*-propyl-2-methoxyphenol can be obtained from the depolymerisation of lignin.

The present invention describes a particular catalytic process for the production of 3-alkylphenol from such 4-alkyl-2-hydroxyphenol or 4-alkyl-2-alkoxyphenol, in which the alkyl substituent is for instance methyl, ethyl or *n*-propyl. This process of present invention comprises the conversion of the 4-alkyl-2-hydroxyphenol or 4-alkyl-2-alkoxyphenol to 3-alkylphenol in the presence of a redox catalyst at elevated temperature under a hydrogen atmosphere. In one embodiment the redox catalyst preferably comprises a noble metal (Pt, Ru, ...) or base metal (Ni, Cu,...) supported on a support of which a considerable part is composed of TiO₂. In yet a more preferred embodiment, the TiO₂ support is composed of the anatase crystal phase. The metal loading on the support ranges from 0.1 to 6 wt%. The reaction is preferably carried out at temperatures between 200 °C and 400 °C and with an hydrogen pressures between 0.5 bar and 100 bar.

An embodiment of present invention describes a process for the production of 3-alkylphenol from such 4-alkyl-2-hydroxyphenol or 4-alkyl-2-alkoxyphenol, in which the alkyl substituent is for instance methyl, ethyl or *n*-propyl. This process of present invention comprises the conversion of the 4-alkyl-2-hydroxyphenol or 4-alkyl-2-alkoxyphenol to 3-alkylphenol at elevated temperature in the presence of a redox catalyst and a hydrogen donating species. In this embodiment the reaction is preferably carried out at temperatures between 200 °C and 400 °C.

Another particular embodiment concerns a catalytic process of selectively forming meta-alkyl phenol from 4-alkyl-2-hydroxyphenol or 4-alkyl-2-alkoxyphenol comprising: supplying a) a 4-alkyl-2-hydroxyphenol or 4-alkyl-2-alkoxyphenol in fluid form and b) a redox catalyst into a reaction vessel or a continuous reactor setup under hydrogen pressure and energizing or heating such reaction medium thus inducing conversion of 4-alkyl-2-hydroxyphenol or 4-alkyl-2-alkoxyphenol to meta-alkyl phenols. The fluid form a 4-alkyl-2-hydroxyphenol or 4-alkyl-2-alkoxyphenol can be a gas fluid or a liquid form. The 4-alkyl-2-hydroxyphenol or 4-alkyl-2-alkoxyphenol can be solved in an organic fluid. In this embodiment the redox catalyst preferably comprises a noble metal (Pt, Ru, ...) or base metal (Ni, Cu,...) supported on a support of which a considerable part is composed of TiOz. In the preferred embodiment, the TiOz support is composed of the anatase crystal phase. The metal loading on the support ranges from 0.1 to 6 wt%. The reaction is preferably carried out at temperatures between 200 °C and 400 °C and hydrogen pressures between 0.5 bar and 100 bar.

Another particular embodiment of present invention concerns a catalytic process of selectively forming meta-alkyl phenol from 4-alkyl-2-hydroxyphenol or4-alkyl-2-alkoxyphenol comprising comprising: supplying a) a 4-alkyl-2-hydroxyphenol or 4-alkyl-2-alkoxyphenol comprising, b) a hydrogen donating solvent and c) a redox catalyst into a reaction vessel and energizing or heating such reaction medium thus inducing conversion of 4-alkyl-2-hydroxyphenol or 4-alkyl-2-alkoxyphenol to a meta-alkyl phenols. In this embodiment the redox catalyst preferably comprises a noble metal (Pt, Ru, ...) or base metal (Ni, Cu,...) supported on a support of which a considerable part is composed of TiOz. In the preferred embodiment, the TiOz support is composed of the anatase crystal phase. The metal loading on the support ranges from 0.1 to 6 wt%. The reaction is preferably carried out at temperatures between 200 °C and 400 °C.

Another particular embodiment of present invention concerns a catalytic process of selectively forming meta-alkyl phenol from 4-alkyl-2-hydroxyphenol or 4-alkyl-2-alkoxyphenol comprising comprising: supplying a) a solvent medium, b) a 4-alkyl-2-hydroxyphenol or 4-alkyl-2-alkoxyphenol, c) a hydrogen donating species and d) a redox catalyst into a reaction vessel and energizing or heating such reaction medium thus inducing conversion of 4-alkyl-2-hydroxyphenol or 4-alkyl-2-alkoxyphenol to a meta-alkyl phenols. In this embodiment the redox catalyst preferably comprises a noble metal (Pt, Ru, ...) or base metal (Ni, Cu,...) supported on a support of which a considerable part is composed of TiOz. In the preferred embodiment, the TiOz support is composed of the anatase crystal phase. The metal loading on the support ranges from 0.1 to 6 wt%. The reaction is preferably carried out at temperatures between 200 °C and 400 °C.

### The present invention relatesto a catalytic process according to claim 1

Some of the techniques described above may be embodied as a catalytic process according to any one of these previous embodiments, whereby the support for the metal is a carbon support or inorganic support, whereby the support comprises titanium dioxide anatase whereon a redox metal. According to the invention, the process iscatalysed by a redox catalyst containing a support that comprises anatase titanium dioxide and a metal on said support in the range of between 0.1 and 6 wt%. In more specific embodiments thereof the TiOz is or comprises crystallized as nano-crystalline anatase and/or the TiOz is or comprises nanotubes in anatase phase and/or the TiOz has hollow structures, for instance in the form of hollow TiOz shells or of mesoporous TiO₂ and/or the TiOz is crystallized as nano-crystalline anatase powder with high specific surface area, up to 250 m²/g, preferably up to 335 m²/g and/or the catalyst comprises of a redox metal on a support, with the support mainlycomprisingTiOz, which is at least for a considerable part in the anatase phase and/or titanium dioxide includes at least 80 wt. % anatase phase, and most preferably a bout 82.8 wt. % to 100 wt. % anatase phase and/or the metal component is selected from Co, Ni, Ru, Rh, Pd, Re, Os, Ir, Pt, Cu and/or mixtures and/or combinations thereof or the metal component is selected from Pd, Pt, Ru, Ni, Cu, and/or mixtures and/or combinations thereof or the metal component is selected from the group consisting of P, V, Sn, Cu, Ni, Co, and Fe and/or mixtures and/or combinations thereof or yet more specific the metal component is comprises a noble metal such as Pt and Ru or a base metal such as Ni and Cu and/or the carbon support or inorganic support component comprises at least one of Al₂O₃, SiOz, ZrOz, and/or mixtures and/or combinations thereof and/or the carbon support or inorganic support comprises a semi-conductive material such as TiOz, ZnO, CeOz, ZrOz, SrTiOs, CaTiOs, and BaTiOs, or mixtures thereof (e.g., composite semiconductors such as TiO_{2/}CeO₂, TiO_{2/}ZrO₂) and/or the carbon support or inorganic support comprises a metal oxide or an alloy oxide of titanium alloyed with iron, aluminium, vanadium, or molybdenum.

Some of the techniques described above may be embodied with the specific features that the amount of the carbon support or inorganic support component is in a range from 1 wt % to 50 wt % of the total weight of the catalyst.

Some of the catalytic process described above may be also embodied with the specific features that the carbon support or inorganic support component comprises Al2O3 and/or SiO₂.

Some of the catalytic process described above may be also embodied with the specific features that the inorganic oxide has a micro structure in which a crystalline phase and an amorphous phase are present together.

Some of the catalytic process described above may be also embodied with the specific features that the amount of the carbon support or inorganic support component is in a range from 1 wt % to 50 wt % of the total weight of the catalyst.

Some of the catalytic process described above may be also embodied with the specific features that carbon support or inorganic support component has an average particle size in a range from 1 µm to 70 µm.

Some of the catalytic process described above may be also embodied with the specific features that at least 50 wt % of the metal component is distributed on the carbon support or inorganic support component.

Some of the catalytic process described above may be also embodied with the specific features that the catalyst is comprised in a molecular sieve.

Some of the catalytic process described above may be also embodied with the specific features that the to be converted starting material is in a fluid phase.

Some of the catalytic process described above may be also embodied with the specific features that fluid is a liquid or that fluid is a gas.

Some of the catalytic process described above may be also embodied with the specific features that the reaction temperature is in the range between 200°C and 500°C, preferably between 250°C and 400°C.

Some of the catalytic process described above may be also embodied with the specific features that the hydrogen pressure is in the range between 0.5 to 150 bars, preferably between 1 to 100 bars.

### ILLUSTRATIVE EMBODIMENTS OFTHE INVENTION

Sources of lignocellulose biomass include bagasse (sugarcane, sweet sorghum,...), barley straw, wheat straws (wheat, barley, rice,...), stover (corn, milo,...), softwood (spruce, pine,...), hardwood (birch, poplar,...), switch grass (Panicum virgatum) and elephant grass.

### Definitions

3-alkylphenols are defined as structures comprising a phenolic unit with a substituent in respectively the 3- or *meta*-position. 4-alkylphenols are defined as structures comprising a phenolic unit with a substituent in respectively the 4- or *para*-position.

4-alkyl-2-hydroxyphenols and 4-alkyl-2-alkoxyphenols can be obtained through various lignin processing techniques like pyrolysis, acid- or base-catalyzed depolymerization, oxidation, liquefaction or hydroprocessing methods like hydrogenolysis or liquid-phase reforming.

"Product yield" is defined as the molar amount of product formed per molar amount of starting substrate and "Substrate conversion" is defined as the molar amount of substrate converted per molar amount of starting material. Product selectivity is defined as the ratio of the product yield over the substrate conversion. For instance a particular embodiment context "Conversion" means the mole percent of the initial alkylphenol that is reacted to form a different compound, not necessarily all the desired product, while "Yield" is the mole percent of the converted starting material that forms any meta-alkyl phenol (m-alkylphenol) and "Selectivity" is the mole percent of the reaction product formed that is meta-alkyl phenol. For example, if 10 moles of alkylphenol are reacted and 2 moles of alkylphenol are left unchanged, then the conversion is 80 percent. If the reaction product contains 6 moles of meta-alkyl phenol then the yield is 60 percent and the selectivity is 75 percent.

The process can be conducted under hydrogen pressure or in a hydrogen-donating solvent or in the presence of hydrogen-donating species. Under hydrogen pressure means that hydrogen gas is present. Hydrogen-donating solvents or hydrogen-donating species are all species (A) that have the ability to donate hydrogen with transformation into an oxidized form (Aₒₓ).

A → xH₂ + Aₒₓ (with x ≥ 1)

Solvents or species that are known to be able to donate hydrogen are for example alcohols like methanol, ethanol, *iso*propanol, etc., acids like formic acid, acidic acid, etc. or hydrocarbons like 9,10-dihydroanthracene, tetralin, etc.

The molar ratio of formed 3-alkylphenol to 4-alkylphenol is defined as the ratio of the molar amount of the 3-alkylphenol to the molar amount of 4-alkylphenol formed during reaction. Since 3- and 4-alkylphenols usually have very similar boiling points (Fiege, H., Voges, H.-W., Hamamoto, T., Umemura, S., Iwata, T., Miki, H., Fujita, Y., Buysch, H.-J., Garbe, D. and Paulus, W. 2000. Phenol Derivatives. Ullmann's Encyclopedia of Industrial Chemistry; Fiege, H. 2000. Cresols and Xylenols. Ullmann's Encyclopedia of Industrial Chemistry), a derivatisation treatment might be required to separate the alkylphenols during gas chromatographic analyses (F. Orata, in Advanced GasChromatography- Progress in Agricultural, Biomedical and Industrial Applications (Ed.: M. A. Mohd), Intech, 2012).

Next to 3-alkylphenol and 4-alkylphenol, also other products like methylated alkylphenols, alkylcyclohexanones, alkylcyclohexane and alkylbenzene are obtained in the reaction. A mixture of 3- and 4-alkylphenol can be isolated via distillation, column chromatography or other separation processes and 3- and 4-alkylphenol can further be separated from one another (Fiege, H., Voges, H.-W., Hamamoto, T., Umemura, S., Iwata, T., Miki, H., Fujita, Y., Buysch, H.-J., Garbe, D. and Paulus, W. 2000. Phenol Derivatives. Ullmann's Encyclopedia of Industrial Chemistry; Fiege, H. 2000. Cresols and Xylenols. Ullmann's Encyclopedia of Industrial Chemistry).

A catalyst suitable for present invention can be prepared by deposition of the redox metal on the catalyst support. Various methods are available to deposit a redox metal on a support, like impregnation by soaking, incipient wetness impregnation, precipitation-deposition, ionexchange, gas phase deposition, etc. (Haber, J., Block, J. H. and Delmon, B. 2008. Methods and Procedures for Catalyst Characterization. Handbook of Heterogeneous Catalysis. 3.3.2:1230-1258). After impregnation of the redox metal, the obtained catalyst precursor can undergo several pretreatment steps like drying, calcination and reduction, before use in the reaction.

A redox metal suitable for present invention can be a metal, or a mixture of metals, that is able to generate and donate active hydrogen species. A redox metal comprises a metal selected from the group of Fe, Co, Ni, Cu, Ru, Rh, Pd, Ir, and Pt and mixtures thereof.

Titanium is widely distributed and occurs primarilyinthe mineralsanatase, brookite, ilmenite, perovskite, rutile and titanite (sphene) >Emsley, John (2001). "Titanium". Nature's Building Blocks: An A-Z Guide to the Elements. Oxford, England, UK: Oxford University Press). Anatase (alias octahedrite) TOz is like rutile TOz a more commonly occurring modification of titanium dioxide. Anatase is always found as small, isolated and sharply developed crystals and it crystallizes in the tetragonal system. The common pyramid of anatase, parallel to the faces of which there are perfect cleavages, has an angle over the polar edge of 82°9', the corresponding angle of rutile being 56°52½'. But crystals of anatase can be prepared in laboratories by chemical methods such as sol-gel method. Examples include controlled hydrolysis of titanium tetrachloride (TiCl₄) or titanium alkoxides. The vertical axis of the crystals is longer than in rutile. Other important differences between the physical characters of anatase and rutile are that the former is less hard (5.5-6 vs. 6-6.5 Mohs) and dense (specific gravity about 3.9 vs. 4.2). Also, anatase is optically negative whereas rutile is positive. For comparison, the refraction index (n) in descending order for several materials follows: rutile (n=between 2.61-2.89); anatase (n=between 2.48-2.56); diamond (n=between 2.41-2.43); gahnite (n=between 1.79-1.82); sapphire (n=between 1.75-1.78); cordierite (n=between 1.52-1.58); beta-spodumene (n=between 1.53-1.57); and residual glass (n=between 1.45-1.49). Also for comparison, the birefringence (DELTA n) in descending order for some of the same materials follows: rutile (DELTA n=between 0.25-0.29); anatase (DELTA n=0.073); sapphire (DELTA n=0.008); cordierite (DELTA n=between 0.005-0.017); diamond (DELTA n=0); and gahnite (DELTA n=0). Based on the above data, it can be seen that some of the Ti-containing crystalline phases, and rutile in particular, are among the materials exhibiting some of the highest refractive indices. In addition, it can be seen that some of the Ti-containing crystalline phases, and rutile in particular, have a relatively high birefringence (DELTA n), a result of the anisotropic character of their tetragonal crystal structure.

A catalyst support suitable for present invention is composed for a considerable part (> 80 wt%) of TiOz in the anatase phase. As known by the experts, the concentration of TiO₂ anatase in the support can also be related to the surface concentration to which the reagents have access to, meaning that coating of a non-TiOz support with TiOz anatase, so that the surface of the support is composed for a considerable part of TiOz anatase, is also included.

In the process of the invention, the reaction temperature depends on the nature and the catalytic activity of the specific catalyst, on the thermal stabilityof the specific catalyst and on the boiling point of the solvent. The reaction is usually carried out between 200 and 400 °C, preferably between 275 and 325 °C.

The reaction can be carried out in a suitable solvent which does not deactivate the catalyst, is stable under reaction conditions and does not adversely affect the reaction in any way. The solvent choice depends further on the substrate solubility. Preferred solvents include, but are not restricted to, *n*-heptane, *n*-hexadecane and *n*-propylcyclohexane.

### ABBREVIATONS

| | |
|---|---|
| 4-AG | 4-alkyl-2-methoxyphenol or 4-alkylguaiacol |
| 4-PG | 4-*n*-propyl-2-methoxyphenol or 4-*n*-propylguaiacol |
| 4-EG | 4-ethyl-2-methoxyphenol or 4-ethylguaiacol |
| 4-EC | 4-ethyl-2-hydroxyphenol or 4-ethyl-1,2-dihydroxybenzene or 4-ethylcatechol |
| 5-AG | 5-alkyl-2-methoxyphenol or 5-alkylguaiacol |
| 3-APh | 3- or *m*-alkylphenol |
| 4-APh | 4- or *p*-alkylphenol |
| MeAPh | Methylalkylphenol |
| ACHol/one | alkylcyclohexanol and alkylcyclohexanone |
| MeOACHol | methoxyalkylcyclohexanol |

### EXAMPLE 1

This example illustrates the preparation of the metal on support catalysts. A 3NiTiOzA1 catalyst was prepared by incipient wetness impregnation of 2 g TiOz powder (Alfa Aesar, catalyst support, 150 m²/g, 100% anatase) with 1.5 mL of an aqueous solution of 0.7 M Ni(NO₃)₂.6H₂O (Alfa Aesar), drying the sample at 60 °C for 12 h and reduction of the sample at 500 °C for 1 h under H₂-flow.

### EXAMPLE 2

This example illustrates the derivatisation procedure of the reaction product. Before analysis of the reaction product by gas chromatography, a derivatisation treatment by trimethylsylilation was performed to enable separation of the 3- and 4-alkylphenols. 0.5 mL of the reaction product, 0.5 mL pyridine (Acros, 99+%) and 0.25 mL N-methyl-N-(trimethylsilyl)trifluoroacetamide (Sigma Aldrich, 97+%) were combined in a vial and placed in an oven at 80 °C for 30 min.

### EXAMPLE 3

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiOzA1 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### EXAMPLE 4

609 mg (4 mmol) 4EG (Sigma Aldrich, 98+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiO2A1 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### EXAMPLE 5

609 mg (4 mmol) 4EC (Sigma Aldrich, 98+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiO2A1 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature, diethylether was added to the reactor ensure complete solubilisation of all compounds (4EC is only weakly soluble in hexadecane), and the reaction mixture was analysed. The catalytic results are shown in Table 1.

### EXAMPLE 6

A 3NiTiO2A2 catalyst was prepared by incipient wetness impregnation of 2 g TiOz powder (Sachtleben Chemie GmbH, HOMBIKAT M 311, ~ 300 m²/g, 100% anatase) with 4.2 mL of an aqueous solution of 0.25 M Ni(NO₃)₂.6H₂O (Alfa Aesar), drying the sample at 60 °C for 12 h and reduction of the sample at 500 °C for 1 h under Hz-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiOzAz catalyst. The reactor was flushed with N₂, heated to 300 °C and put under constant H₂ pressure at 2 MPa H₂. The stirrer speed was set at 750 rpm. After 2 h reaction, the reactor was cooled down to room temperature and the reaction mixture was analysed. The catalytic results are shown in Table 1.

### COUNTER EXAMPLE 1

A 3NiZrO2 catalyst was prepared by incipient wetness impregnation of 2 g ZrOz powder (Alfa Aesar, catalyst support, 90 m²/g)with 1.23 mL of an aqueous solution of 0.86 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiZrO2 catalyst. The reactor was flushed with N₂, heated to 300 °C and put under constant H₂ pressure at 2 MPa H₂. The stirrer speed was set at 750 rpm. After 1 h reaction, the reactor was cooled down to room temperature and the reaction mixture was analysed. The catalytic results are shown in Table 1.

### COUNTER EXAMPLE 2

A 3NiTiO₂RU catalyst was prepared by incipient wetness impregnation of 2 g TiOz powder (Sachtleben Chemie GmbH, ~ 100 m²/g, 100% rutile) with 1.92 mL of an aqueous solution of 0.55 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiO2RU catalyst. The reactor was flushed with N₂, heated to 300 °C and put under constant H₂ pressure at 2 MPa H₂. The stirrer speed was set at 750 rpm. After 2 h reaction, the reactor was cooled down to room temperature and the reaction mixture was analysed. The catalytic results are shown in Table 1.

### COUNTER EXAMPLE 3

A 3NiTiO₂ARU catalyst was prepared by incipient wetness impregnation of 2 g TiOz powder (Evonik Industries, Aeroxide^{®} TiO₂ P25, ~ 35-65 m²/g, mixture of anatase and rutile) with 1.9 mL of an aqueous solution of 0.55 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiO2ARU catalyst. The reactor was flushed with N₂, heated to 300 °C and put under constant H₂ pressure at 2 MPa H₂. The stirrer speed was set at 750 rpm. After 1 h reaction, the reactor was cooled down to room temperature and the reaction mixture was analysed. The catalytic results are shown in Table 1.

### EXAMPLE 7

A 0.5RuTiO₂A1 catalyst was prepared by incipient wetness impregnation of 2 g TiOz powder (Alfa Aesar, catalyst support, 150 m²/g, 100% anatase) with 1.5 mL of an aqueous solution of 0.067 M Ru(NH₃)₆Cl₃ (Acros Organics), drying the sample at 60 °C for 12 h, calcination of the sample at 400 °C for 1 h under N₂-flow and reduction of the sample at 400 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 0.5RuTiOzA1 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### EXAMPLE 8

A 0.5PtTiOzA1 catalyst was prepared by incipient wetness impregnation of 2 g TiOz powder (Alfa Aesar, catalyst support, 150 m²/g, 100% anatase) with 1.5 mL of an aqueous solution of 0.018 M Pt(NH₃)₄Cl₂H₂O (Alfa Aesar), drying the sample at 60 °C for 12 h, calcination of the sample at 400 °C for 1 h under Oz-flow and reduction of the sample at 400 °C for 1 h under Hz-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 0.5PtTiOzA1 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### EXAMPLE 9

A 3CuTiO₂A1 catalyst was prepared by incipient wetness impregnation of 2 gTiOz powder (Alfa Aesar, catalyst support, 150 m²/g, 100% anatase) with 1.5 mL of an aqueous solution of 0.24 M Cu(NO₃)₂3H₂O (Sigma Aldrich), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3CuTiOzA1 catalyst. The reactor was flushed with N₂, heated to 300 °C and put under constant H₂ pressure at 2 MPa H₂. The stirrer speed was set at 750 rpm. After 6 h reaction, the reactor was cooled down to room temperature and the reaction mixture was analysed. The catalytic results are shown in Table 1.

### COUNTER EXAMPLE 4

A TiO2A1 catalyst was prepared by drying the TiOz powder (Alfa Aesar, catalyst support, 150 m²/g, 100% anatase) at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the TiO2A1 catalyst. The reactor was flushed with N₂, heated to 300 °C and put under constant H₂ pressure at 2 MPa H₂. The stirrer speed was set at 750 rpm. After 1 h reaction, the reactor was cooled down to room temperature and the reaction mixture was analysed. The catalytic results are shown in Table 1.

### EXAMPLE 10

A 0.5NiTiO2A1 catalyst was prepared by incipient wetness impregnation of 2 g TiOz powder (Alfa Aesar, catalyst support, 150 m²/g, 100% anatase) with 1.5 mL of an aqueous solution of 0.11 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 0.5NiTiO₂A1 catalyst. The reactor was flushed with N2, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### EXAMPLE 11

A 6NiTiO2A1 catalyst was prepared by incipient wetness impregnation of 2 gTiOz powder (Alfa Aesar, catalyst support, 150 m²/g, 100% anatase) with 1.5 mL of an aqueous solution of 1.44 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 6NiTiO₂A1 catalyst. The reactor was flushed with N2, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

### COUNTER EXAMPLE 5

A 12NiTiO₂A1 catalyst was prepared by incipient wetness impregnation of 2 g TiOz powder (Alfa Aesar, catalyst support, 150 m²/g, 100% anatase) with 1.5 mL of an aqueous solution of 3.08 M Ni(NO₃)₂.6H₂O (Alfa Aesar), drying the sample at 60 °C for 12 h and reduction of the sample at 500 °C for 1 h under H₂-flow.

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 12NiTiO₂A1 catalyst. The reactor was flushed with N2, heated to 300 °C and put under constant H₂ pressure at 2 MPa H₂. The stirrer speed was set at 750 rpm. After 1 h reaction, the reactor was cooled down to room temperature and the reaction mixture was analysed. The catalytic results are shown in Table 1.

### EXAMPLE 12

665 mg (4 mmol) 4PG (Sigma Aldrich, 99+%), 100 mg decane (Sigma Aldrich, 99+%, internal standard) and 20 mL hexadecane solvent (Sigma Aldrich, 99%) were placed in a 50 mL autoclave, together with 0.2 g of the 3NiTiO2A1 catalyst. The reactor was flushed with N₂, heated to 300 °C and put under constant H₂ pressure at 6 MPa H₂. The stirrer speed was set at 750 rpm. After 2 h reaction, the reactor was cooled down to room temperature and the reaction mixture was analysed. The catalytic results are shown in Table 1.

### EXAMPLE 13

4000 mg (24 mmol) 4PG (Sigma Aldrich, 99+%), 300 mg decane (Sigma Aldrich, 99+%, internal standard) and 20 mL hexadecane solvent (Sigma Aldrich, 99%) were placed in a 50 mL autoclave, together with 1 g of the 3NiTiO₂A2 catalyst. The reactor was flushed with N₂, heated to 300 °C and put under constant H₂ pressure at 6 MPa H₂. The stirrer speed was set at 750 rpm. After 2 h reaction, the reactor was cooled down to room temperature and the reaction mixture was analysed. The catalytic results are shown in Table 1.

### EXAMPLE 14

665 mg (4 mmol) 4PG (Sigma Aldrich, 99+%), 300 mg decane (Sigma Aldrich, 99+%, internal standard) and 20 mL n-propylcyclohexane solvent (Sigma Aldrich, 99%) were placed in a 50 mL autoclave, together with 1 g of the 3NiTiO₂A2 catalyst. The reactor was flushed with N₂, heated to 300 °C and put under constant H₂ pressure at 2 MPa H₂. The stirrer speed was set at 750 rpm. After 4 h reaction, the reactor was cooled down to room temperature and the reaction mixture was analysed. The catalytic results are shown in Table 1.

| **Table 1** Catalytic results | | | | | | | | | | | | | |
|---|---|---|---|---|---|---|---|---|---|---|---|---|---|
| | **Catalyst** | **Substrate** | **Conv. (%)** | | **Yield (%)** | | | | | | | | **3-APh/4-APh ratio** |
| | | | | | 5-AG | 3-APh | 4-APh | MeAPh | ACHoI/one | MeOACHol | Alkanes & aromatics | | |
| Exp. 3 | 3NiTiO2A1 | 4PG | **98.1** | | 2.2 | 59.5 | 11.7 | 20.5 | 1.3 | 0 | 0.5 | | 5.1 |
| Exp. 4 | 3NiTiO2A1 | 4EG | **98.3** | | 1.7 | 53.5 | 10.8 | 16.6 | 1.2 | 0 | 0 | | 5 |
| Exp. 5 | 3NiTiO2A1 | 4EC | **98.5** | | / | 75.4 | 21.6 | / | 1.8 | / | 0.4 | | 3.5 |
| Exp. 6 | 3NiTiO2A2 | 4PG | **97.1** | | 2.3 | 57.5 | 11.1 | 19.9 | 1.9 | 0 | 0.2 | | 5.2 |
| Count. Exp. 1 | 3NiZrO2 | 4PG | **98.8** | | 0 | 0.4 | 0.4 | 0.6 | 55.2 | 0 | 34.5 | | 1.1 |
| Count. Exp. 2 | 3NiTiO2RU | 4PG | **95.1** | | 2.3 | 2.1 | 4.2 | 1.7 | 66.5 | 5.6 | 7.4 | | 0.5 |
| Count. Exp. 3 | 3NiTiO2ARU | 4PG | **94.2** | | 0.6 | 1.2 | 0.9 | 3.5 | 25.6 | 0 | 56.4 | | 1.3 |
| Exp. 7 | 0.5RuTiO2A1 | 4PG | **98.2** | | 3.1 | 59.8 | 11.5 | 17.2 | 0.9 | 0 | 0 | | 5.2 |
| Exp. 8 | 0.5PtTiO2A1 | 4PG | **99.1** | | 0.9 | 56.4 | 11.3 | 18.4 | 2.1 | 0 | 0 | | 5.0 |
| Exp. 9 | 3CuTiO2A1 | 4PG | **99.2** | | 0.8 | 56.3 | 11.1 | 16.2 | 0.3 | 0 | 0 | | 5.1 |
| Count. Exp. 4 | TiO2A1 | 4PG | **12** | | 3.8 | 1.4 | 0.6 | 0.4 | 0 | 0 | 0 | | 2.33 |
| Exp. 10 | 0.5NiTiO2A1 | 4PG | **97.9** | | 2.1 | 58.8 | 11.7 | 17.8 | 0.6 | 0 | 0 | | 5 |
| Exp. 11 | 6NiTiO2A1 | 4PG | **98.5** | | 1.9 | 58.2 | 11.9 | 19.2 | 1.1 | 0 | 0 | | 4.9 |
| Count. Exp. 5 | 12NiTiO2A1 | 4PG | **91.4** | | 1.9 | 8.8 | 6.0 | 4.3 | 28.5 | 0.7 | 35.5 | | 1.5 |
| Exp. 12 | 3NiTiO2A1 | 4PG | **98.5** | | 1.6 | 58.3 | 11.4 | 18.5 | 2.2 | 0 | 0 | | 5.1 |
| Exp. 13 | 3NiTiO2A2 | 4PG | **97.4** | | 2.1 | 56.5 | 11.8 | 16.4 | 5.6 | 0 | 6.3 | | 4.8 |
| Exp. 14 | 3NiTiO2A2 | 4PG | **98.3** | | 1.5 | 54.7 | 10.5 | 17.2 | 0.8 | 0 | / | | 5.2 |

## Claims

1. A catalytic process for converting a 4-R₂-2-R₁O-phenol for instance a 4-alkyl-2-hydroxyphenol or a 4-alkyl-2-alkoxyphenol (e.g. 4-*n*-propyl-2-hydroxyphenol, 4-ethyl-2-hydroxyphenol, 4-*n*-propyl-2-methoxyphenol, 4-ethyl-2-methoxyphenol or 4-n-propyl-2-ethoxyphenol), comprising: supplying 1) said 4-R₂-2-R₁O-phenol and 2) a hydrogen donating solvent, species or a hydrogen atmosphere and 3) a redox catalyst comprising anatase titanium dioxide (TiOz) and redox metal into a reaction vessel and 3) energizing or heating such medium thus inducing conversion of 4-alkyl-2-hydroxyphenol or 4-alkyl-2-alkoxyphenol into a 3-alkylphenol, with the 4-alkyl-2-alkoxyphenol or 4-alkyl-2-hydroxyphenol having one of the following structure: 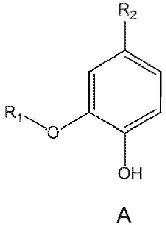
a. wherein in structure A (phenolic monomer) R₁ is H or CₙH₂ₙ₊₁ with n = 1, 2 or 3 and R₂ is CₙH₂ₙ₊₁ with n = 1, 2, 3 or 4 or CₙH₂ₙOH with n = 1, 2, 3 or 4 or CₙH₂ₙ₋₁O with n = 1, 2, 3 or 4 or CₙH₂ₙ₋₁ with n = 2, 3 or 4,
wherein the process is catalysed by a redox catalyst containing a support that comprises anatase titanium dioxide and a metal on said support in the range of between 0.1 and 6 wt%, and
wherein said titanium dioxide includes at least 80 wt.% anatase phase.

2. The catalytic process according to claim 1, whereby the support for the metal is a carbon support or inorganic support, whereby the support comprises titanium dioxide anatase whereon a redox metal.

3. The catalytic process according to any one of the claims 1 to 2, whereby the catalyst comprises of a redox metal on a support, with the support mainly comprising TiO₂, which is at least for a considerable part in the anatase phase.

4. The catalytic process according to any one of the claims 1 to 3, whereby the metal component is selected from Co, Ni, Ru, Rh, Pd, Re, Os, Ir, Pt, Cu and/or mixtures and/or combinations thereof.

5. The catalytic process according to any one of the claims 1 to 3, whereby the metal component is selected from the group consisting of P, V, Sn, Cu, Ni, Co, and Fe and/or mixtures and/or combinations thereof.

6. The catalytic process according to any one of the claims 2 to 5, whereby the carbon support or inorganic support component comprises at least one of Al₂O₃, SiO₂, ZrO₂, and/or mixtures and/or combinations thereof.

7. The catalytic process according to claim 6, whereby the inorganic oxide has a micro structure in which a crystalline phase and an amorphous phase are present together.

8. The catalytic process according to any one of the claims 2 to 7, whereby the amount of the carbon support or inorganic support component is in a range from 1 wt % to 50 wt % of the total weight of the catalyst.

9. The catalytic process according to any one of the claims 2 to 8, whereby carbon support or inorganic support component has an average particle size in a range from 1 µm to 70 µm.

10. The catalytic process according to any one of the claims 1 to 9, whereby the to be converted starting material is in a fluid phase, whereby the fluid is of the group consisting of a liquid or a gas.

11. The process according to any one of the previous claims 1 to 10, wherein the reaction temperature is in the range between 200°C and 500°C, preferably between 250°C and 400°C.

12. The process according to any one of the previous embodiments 1 to 11, wherein the hydrogen pressure is in the range between 0.5 to 150 bars, preferably between 1 to 100 bars.

## Patentansprüche

1. Katalytisches Verfahren zum Umwandeln eines 4-R₂-2-R₁O-Phenols, beispielsweise eines 4-Alkyl-2-hydroxyphenols oder eines 4-Alkyl-2-Alkoxyphenols (z.B. 4-*n*-Propyl-2-hydroxyphenols, 4-Ethyl-2-hydroxyphenols, 4-*n*-Propyl-2-methoxyphenols, 4-Ethyl-2-methoxyphenols oder 4-*n-*Propyl-2-ethoxyphenols) umfassend: Liefern 1) des 4-R₂-2-R₁O-Phenols und 2) eines Wasserstoff spendenden Lösungsmittels, einer Wasserstoff spendenden Spezies oder einer Wasserstoffatmosphäre und 3) eines Redoxkatalysators umfassend Anatas-Titandioxid (TiO₂) und Redoxmetall in ein Reaktionsgefäß und 3) Energetisieren oder Erhitzen eines derartigen Mediums und so Herbeiführen der Umwandlung von 4-Alkyl-2-hydroxyphenol oder 4-Alkyl-2-alkoxyphenol in ein 3-Alkylphenol, wobei das 4-Alkyl-2-alkoxyphenol oder 4-Alkyl-2-hydroxyphenol eine der folgenden Struktur aufweist: 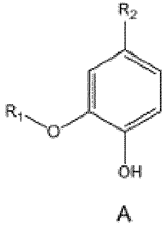
a. wobei in der Struktur A (phenolischem Monomer) R₁ H oder CₙH₂ₙ₊₁, wobei n = 1, 2 oder 3, ist und R₂ CₙH₂ₙ₊₁, wobei n = 1, 2, 3 oder 4, oder CₙH₂ₙOH, wobei n = 1, 2, 3 oder 4, oder CₙH₂ₙ₊₁O, wobei n = 1, 2, 3 oder 4, oder CₙH₂ₙ₋₁, wobei n = 2, 3 oder 4, ist,
wobei das Verfahren durch einen Redoxkatalysator katalysiert wird, der einen Träger enthält, der Anatas-Titandioxid und ein Metall auf dem Träger im Bereich zwischen 0,1 und 6 Gew.-% umfasst und
wobei das Titandioxid mindestens 80 Gew.-% Anatasphase umfasst.

2. Katalytisches Verfahren nach Anspruch 1, wobei der Träger für das Metall ein Kohlenstoffträger oder anorganischer Träger ist, wobei der Träger Titandioxid-Anatas umfasst, auf dem ein Redoxmetall.

3. Katalytisches Verfahren nach einem der Ansprüche 1 bis 2, wobei der Katalysator ein Redoxmetall auf einem Träger umfasst, wobei der Träger hauptsächlich TiO₂ umfasst, das sich mindestens zu einem beträchtlichen Teil in der Anatasphase befindet.

4. Katalytisches Verfahren nach einem der Ansprüche 1 bis 3, wobei die Metallkomponente unter Co, Ni, Ru, Rh, Pd, Re, Os, Ir, Pt, Cu und/oder Mischungen und/oder Kombinationen davon ausgewählt ist.

5. Katalytisches Verfahren nach einem der Ansprüche 1 bis 3, wobei die Metallkomponente aus der Gruppe ausgewählt ist bestehend aus P, V, Sn, Cu, Ni, Co und Fe und/oder Mischungen und/oder Kombinationen davon.

6. Katalytisches Verfahren nach einem der Ansprüche 2 bis 56, wobei der Kohlenstoffträger oder die anorganische Trägerkomponente mindestens eines umfasst von Al₂O₃, SiO₂, ZrO₂ und/oder Mischungen und/oder Kombinationen davon.

7. Katalytisches Verfahren nach Anspruch 6, wobei das anorganische Oxid eine Mikrostruktur aufweist, in der eine kristalline Phase und eine amorphe Phase zusammen vorliegen.

8. Katalytisches Verfahren nach einem der Ansprüche 2 bis 7, wobei die Menge des Kohlenstoffträgers oder der anorganischen Trägerkomponente in einem Bereich von 1 Gew.-% bis 50 Gew.-% des Gesamtgewichts des Katalysators liegt.

9. Katalytisches Verfahren nach einem der Ansprüche 2 bis 8, wobei der Kohlenstoffträger oder die anorganische Trägerkomponente eine durchschnittliche Teilchengröße in einem Bereich von 1 µm bis 70 µm aufweist.

10. Katalytisches Verfahren nach einem der Ansprüche 1 bis 9, wobei das umzuwandelnde Ausgangsmaterial sich in einer fluiden Phase befindet, wobei das Fluid zu der Gruppe gehört bestehend aus einer Flüssigkeit oder einem Gas.

11. Verfahren nach einem der vorhergehenden Ansprüche 1 10, wobei die Reaktionstemperatur im Bereich zwischen 200 °C und 500 °C, bevorzugt zwischen 250 °C und 400 °C, liegt.

12. Verfahren nach einem der vorhergehenden Ansprüche 1 bis 11, wobei der Wasserstoffdruck im Bereich zwischen 0,5 und 150 bar, bevorzugt zwischen 1 und 100 bar, liegt.

## Revendications

1. Processus catalytique pour transformer un 4-R₂-2-R₁O-phénol, par exemple un 4-alkyl-2-hydroxyphénol ou un 4-alkyl-2-alkoxyphénol (par exemple du 4-n-propyl-2-hydroxyphénol, 4-éthyl-2-hydroxyphénol, 4-n-propyl-2-méthoxyphénol, 4-éthyl-2-méthoxyphénol ou 4-*n-*propyl-2-éthoxyphénol), comprenant : la fourniture 1) dudit 4-R₂-2-R₁O-phénol et 2) d'un solvant donnant de l'hydrogène, une espèce chimique ou une atmosphère d'hydrogène et 3) d'un catalyseur d'oxydo-réduction comprenant du dioxyde de titane anatase (TiO₂) et un métal d'oxydo-réduction dans un récipient de réaction et 3) la stimulation ou le chauffage d'un tel milieu induisant ainsi la transformation de 4-alkyl-2-hydroxyphénol ou de 4-alkyl-2-alkoxyphénol en un 3-alkylphénol, le 4-alkyl-2-alkoxyphénol ou le 4-alkyl-2-hydroxyphénol ayant l'une de la structure suivante : 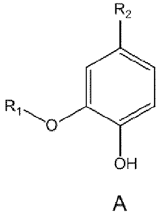
a. dans laquelle dans la structure A (monomère phénolique) R₁ est H ou CₙH₂ₙ₊₁ avec n = 1, 2 ou 3 et R₂ est CₙH₂ₙ₊₁ avec n = 1, 2, 3 ou 4 ou CₙH₂ₙOH avec n = 1, 2, 3 ou 4 ou CₙH2ₙ₋₁O avec n = 1, 2, 3 ou 4 ou CₙH₂ₙ₋₁ avec n = 2, 3 ou 4,
dans lequel le processus est catalysé par un catalyseur d'oxydo-réduction contenant un support qui comprend du dioxyde de titane anatase et un métal sur ledit support dans la plage entre 0,1 et 6 % en poids, et
dans lequel ledit dioxyde de titane inclut au moins 80 % en poids de phase anatase.

2. Processus catalytique selon la revendication 1, par lequel le support pour le métal est un support carbonique ou un support inorganique, par lequel le support comprend du dioxyde de titane anatase sur lequel il y a un métal d'oxydo-réduction.

3. Processus catalytique selon l'une quelconque des revendications 1 à 2, par lequel le catalyseur comprend un métal d'oxydo-réduction sur un support, le support comprenant principalement du TiO₂, qui est au moins pour une partie considérable dans la phase anatase.

4. Processus catalytique selon l'une quelconque des revendications 1 à 3, par lequel le composant métallique est sélectionné parmi Co, Ni, Ru, Rh, Pd, Re, Os, Ir, Pt, Cu et/ou des mélanges et/ou des combinaisons de ceux-ci.

5. Processus catalytique selon l'une quelconque des revendications 1 à 3, par lequel le composant métallique est sélectionné dans le groupe constitué par P, V, Sn, Cu, Ni, Co et Fe et/ou des mélanges et/ou des combinaisons de ceux-ci.

6. Processus catalytique selon l'une quelconque des revendications 2 à 5, par lequel le composant de support carbonique ou de support inorganique comprend au moins l'un de Al₂O₃, SiO₂, ZrO₂, et/ou de mélanges et/ou de combinaisons de ceux-ci.

7. Processus catalytique selon la revendication 6, par lequel l'oxyde inorganique a une microstructure dans laquelle une phase cristalline et une phase amorphe sont présentes ensemble.

8. Processus catalytique selon l'une quelconque des revendications 2 à 7, par lequel la quantité du composant de support carbonique ou de support inorganique est dans une plage de 1 % en poids à 50 % en poids du poids total du catalyseur.

9. Processus catalytique selon l'une quelconque des revendications 2 à 8, par lequel le composant de support carbonique ou de support inorganique a une taille de particule moyenne dans une plage allant de 1 µm à 70 µm.

10. Processus catalytique selon l'une quelconque des revendications 1 à 9, par lequel la matière de départ à transformer est dans une phase liquide, par lequel le fluide est du groupe consistant en un liquide ou en un gaz.

11. Processus selon l'une quelconque des revendications 1 à 10, dans lequel la température de réaction est dans la plage entre 200 °C et 500 °C, de préférence entre 250 °C et 400 °C.

12. Processus selon l'un quelconque des modes de réalisation précédents 1 à 11, dans lequel la pression d'hydrogène est dans la plage entre 0,5 et 150 bars, de préférence entre 1 et 100 bars.
